# EUROPEAN PATENT APPLICATION

(11) **EP 3 498 697 A1**
(43) Date of publication of application: **19.06.2019**
(21) Application number: 17382841.9
(22) Date of filing: 12.12.2017
(51) Int. Cl.: C07D 249/12, A61K 31/4196, A61P 9/00, A61P 29/00

(54) **NOVEL SALTS AND POLYMORPHS OF LESINURAD**

(71) Applicant: Química Sintética, S.A., 08028 Barcelona (ES)
(72) Inventor: Barreca, Giuseppe, 23874 MONTEVECCHIA (LC) (IT); Ventimiglia, Gianpiero, 72021 FRANCAVILLA FONTANA (BR) (IT); Marras, Giovanni, 28066 GALLIATE (NO) (IT)
(74) Representative: ZBM Patents - Zea, Barlocci & Markvardsen

(57) **Abstract**

New salts and polymorphs of lesinurad, having the formula (I) reported below, are provided.

Also described are the processes for the preparation of said solid forms.

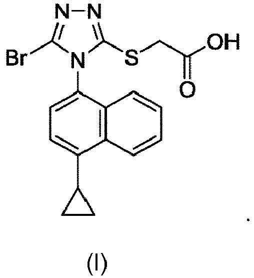

## Description

### Technical Field

The present invention relates to novel salts and polymorphs of lesinurad, to pharmaceutical compositions comprising them as well as to the processes for their preparation, and methods of their use.

### Background Art

Lesinurad is the INN denomination assigned to the compound having IUPAC name 2-(5-bromo-4-(4-cyclopropylnaphthalen-1-yl)-4H-1,2,4-triazol-3-ylthio)acetic acid and the formula reported below:

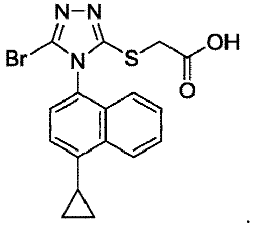

Lesinurad inhibits the uric acid transporter 1 (URAT1) protein, increasing uric acid excretion and thereby lowering serum uric acid, and is used in combination with a xanthine oxidase inhibitor such as allopurinol or febuxostat for the treatment of gout in patients affected by hyperuricemia.

The family of compounds to which lesinurad belongs is disclosed in patent application WO 2006/026356 A2, while patent applications WO 2009/070740 A2, WO 2014/008295 A1 and WO 2014/198241 A1 are directed to processes for the preparation of these compounds.

As generally known, any active pharmaceutical ingredient may exist under amorphous or different crystalline forms, either as pure compound or in forms in which, in the structure of the crystal, are present molecules of water (hydrates) or of another solvent (solvates); besides, in case of hydrates and solvates, the ratio between the number of molecules of active principle and molecules of water or solvent may vary, giving rise to different solid forms of the compound.

Different salts and solid-state forms of an active pharmaceutical ingredient may possess different properties (e.g., density, compressibility, hardness, morphology, cleavage, stickiness, solubility, water uptake, electrical properties, thermal behaviour, solid-state reactivity, physical stability, and chemical stability) which can be determined using analytical chemical techniques, including solid-state analytical techniques (e.g., X-ray diffraction, microscopy, spectroscopy and thermal analysis), as described herein and known in the art.

Such variations in the properties of different salts and solid-state forms may provide a basis for improving formulation, for example, by facilitating better processing or handling characteristics, changing the dissolution profile in a favourable direction, or improving stability (polymorphic and/or chemical) and shelf-life. These variations in the properties of different salts and solid-state forms may also offer improvements to the final dosage form, for instance, if they serve to improve bioavailability.

Different salts, solid-state forms and solvates of an active pharmaceutical ingredient may also give rise to a variety of polymorphs or crystalline forms, which, in turn, may provide additional opportunities to assess variations in the properties and characteristics of a solid active pharmaceutical ingredient.

For these reasons, chemical compounds useful in the pharmaceutical field are systematically screened looking for the physical form(s) that present the best set of production, storage and handling properties, and which are best-suited for administration to patients.

Patent application WO 2012/092395 A2 discloses two crystalline forms of lesinurad, referred to in the document respectively as Form 1 and Form 2. Form 1 is characterized by an X-ray powder diffraction pattern having the main peaks at about 10.32°, 18.84°, 20.75°, 21.54°, 27.28 2θ, while Form 2 is characterized by an X-ray powder diffraction pattern having the main peaks at about 10.46°, 11.96°, 13.82°, 16.19°, 18.21°, 19.83° 2θ.

Patent application WO 2015/075561 A2 provides crystalline forms of lesinurad, designated as Forms III, IV, V, VI, respectively. Form III is a non-solvated form characterized by an X-ray powder diffraction pattern having the main peaks at about 7.95°, 11.93°, 17.36°, 20.85°, 23.76°, 27.24° 2θ. Form IV is a dichloromethane solvate characterized by an X-ray powder diffraction pattern having the main peaks at about 6.83°, 18.54°, 24.10°, 24.56°, 26.73° 2θ. Form V is a 2-methyl tetrahydrofuran solvate characterized by an X-ray powder diffraction pattern having the main peaks at about 6.12°, 18.70°, 20.10°, 20.88°, 24.84°, 26.21° 2θ. Form VI is a trichloromethane solvate characterized by an X-ray powder diffraction pattern having the main peaks at about 6.64°, 17.94°, 18.27°, 21.28°, 23.47°, 27.68° 2θ.

An object of the present invention was the provision of novel polymorphic forms of lesinurad allowing an almost complete removal of the impurities resulting from its synthesis, and enabling preparation of a high-purity products containing amounts of possibly harmful compounds as low as possible.

### Summary of the invention

These objectives are achieved with the present invention that, in a first aspect thereof, relates to novel crystalline forms of 2-(5-bromo-4-(4-cyclopropylnaphthalen-1-yl)-4H-1,2,4-triazol-3-ylthio)acetic acid (lesinurad) or a salt thereof.

In a second aspect thereof, the invention relates to the processes for producing the crystalline forms of object of the first aspect of the invention.

The present invention also relates to solid pharmaceutical compositions comprising, as active pharmaceutical ingredient, an effective amount of the crystalline forms object of the first aspect of the invention, as well as to said crystalline forms or pharmaceutical compositions comprising them for use in therapy.

### Brief description of the drawings

Figure 1 depicts the X-Ray powder diffractogram of lesinurad crystalline form F.
Figure 2 depicts the X-Ray powder diffractogram of lesinurad crystalline form J.
Figure 3 depicts the X-Ray powder diffractogram of lesinurad crystalline form K.
Figure 4 depicts the X-Ray powder diffractogram of lesinurad dicyclohexylamine salt.
Figure 5 depicts the X-Ray powder diffractogram of lesinurad 4-methylmorpholine salt.

### Detailed description of the invention

All terms used in this application, unless otherwise specified, are to be understood in their ordinary meaning as known in the technical field.

The term "*about*" includes the range of experimental errors, which can normally occur performing a measurement.

The term "*seed*" refers to a crystalline substance that is added to a dispersion of the same substance to induce its crystallization. Seeding with a specific crystalline form has often the useful effect of promoting crystallization of the substance in the same crystalline form of the seed.

The term "mass" defines the combination of substrates, reagents, solvents, and products on which a physical or chemical transformation is carried out.

In general, solvates of a crystalline solid include one or more molecule of solvent in the interstices or voids of the crystal network. The filling of the voids of the crystalline structure with one or more solvent species (e.g. acetic or formic acid) takes place according to the molar volume of the guest molecule and the empty space of the host structure, also taking into account the possibility to form a network of hydrogen bonding.

The term "*solvate*" refers to a molecular complex comprising lesinurad or a derivative thereof and a stoichiometric or non-stoichiometric amount of a solvent, e.g., acetic acid or formic acid.

By "*polymorphically stable*" it is meant that the crystalline forms of lesinurad when stored under stressed conditions (i.e., in the case of crystalline form F, up to a temperature of 35 °C under *vacuum* for 24 hours; in the case of crystalline form K, up to a temperature of 50 °C under *vacuum* for 24 hours or, in the case of crystalline form J, up to a temperature of 80 °C), show no signs of crystallinity associated to other polymorphs of lesinurad as judged by the absence of their relevant peaks in an X-ray powder diffractogram (XRPD).

In its first aspect, the invention relates to novel crystalline forms of lesinurad or a salt thereof.

According to a first embodiment of this aspect of the invention, a polymorphically stable crystalline form of lesinurad (referred to in the rest of description as "form J") is provided. Said form J shows an X-ray powder diffraction pattern that, when collected with the Kα radiation of copper (λ = 1.5408 Å), is characterized by peaks at:
i. 9.32°, 15.88°, 18.20° and 26.68° ± 0.2° 2θ; or
ii. 9.32°, 10.20°, 15.88°, 18.20°, 18.64°, 22.24°, 22.52°, 23.12°, 25.92° and 26.68° ± 0.2° 2θ.

Preferably, said polymorphically stable crystalline form J is substantially free of residual solvents (as determined by, e.g., ¹H NMR analysis), and/or an anhydrous form, as determined by, e.g., Karl Fischer titration.

More preferably said crystalline form J shows an X-ray powder diffraction pattern that, when collected with the Kα radiation of copper (λ = 1.5408 Å), is characterized by at least 4 peaks (± 0.2° 2θ) selected from Table 1A or 1B:

| **Table 1A** | | |
|---|---|---|
| **° 2θ** | ***d* space (Å)** | **Iᵣₑₗ (%)** |
| 9.32 | 9.4814 | 74 |
| 10.20 | 8.6653 | 79 |
| 11.68 | 7.5704 | 38 |
| 14.68 | 6.0294 | 37 |
| 15.88 | 5.5764 | 17 |
| 18.20 | 4.8704 | 97 |
| 18.64 | 4.7564 | 60 |
| 20.52 | 4.3247 | 44 |
| 21.60 | 4.1109 | 35 |
| 22.24 | 3.9940 | 50 |
| 22.52 | 3.9450 | 63 |
| 23.12 | 3.8439 | 53 |
| 25.56 | 3.4822 | 47 |
| 25.92 | 3.4347 | 54 |
| 26.68 | 3.3385 | 100 |

| **Table 1B** | | |
|---|---|---|
| **° 2θ** | ***d* space (Å)** | **Iᵣₑₗ (%)** |
| 9.32 | 9.4814 | 74 |
| 10.20 | 8.6653 | 79 |
| 11.68 | 7.5704 | 38 |
| 12.88 | 6.8677 | 26 |
| 14.68 | 6.0294 | 37 |
| 15.88 | 5.5764 | 17 |
| 16.60 | 5.3361 | 16 |
| 18.20 | 4.8704 | 97 |
| 18.64 | 4.7564 | 60 |
| 20.52 | 4.3247 | 44 |
| 21.60 | 4.1109 | 35 |
| 22.24 | 3.9940 | 50 |
| 22.52 | 3.9450 | 63 |
| 23.12 | 3.8439 | 53 |
| 24.20 | 3.6748 | 20 |
| 24.84 | 3.5815 | 25 |
| 25.56 | 3.4822 | 47 |
| 25.92 | 3.4347 | 54 |
| 26.68 | 3.3385 | 100 |
| 28.28 | 3.1532 | 21 |
| 30.40 | 2.9379 | 21 |
| 32.00 | 2.7946 | 29 |
| 33.60 | 2.6651 | 25 |
| 34.52 | 2.5961 | 22 |
| 35.44 | 2.5308 | 15 |
| 37.04 | 2.4251 | 16 |
| 39.24 | 2.2941 | 16 |

A second embodiment of this aspect of the invention provides for a polymorphically stable crystalline form of lesinurad (referred to in the rest of description as "form K") which is an acetic acid solvate as determined by, e.g., ¹H NMR analysis.

Preferably said crystalline form K shows an X-ray powder diffraction pattern that, when collected with the Kα radiation of copper (λ = 1.5408 Å), is characterized by peaks at:
iii. 7.88°, 10.96°, 19.32° and 28.36° ± 0.2° 2θ; or
iv. 7.88°, 10.96°, 19.32°, 23.76°, 25.00° and 28.36° ± 0.2° 2θ.

More preferably the crystalline form K shows an X-ray powder diffraction pattern that, when collected with the Kα radiation of copper (λ = 1.5408 Å), is characterized by at least 4 peaks (± 0.2° 2θ) selected from Table 2A or 2B:

| **Table 2A** | | |
|---|---|---|
| **° 2θ** | ***d* space (Å)** | **Iᵣₑₗ (%)** |
| 7.88 | 11.2106 | 100 |
| 10.96 | 8.0661 | 21 |
| 13.12 | 6.7426 | 36 |
| 14.72 | 6.0131 | 38 |
| 18.60 | 4.7666 | 40 |
| 19.32 | 4.5905 | 88 |
| 20.12 | 4.4098 | 42 |
| 21.60 | 4.1109 | 37 |
| 22.00 | 4.0370 | 40 |
| 22.56 | 3.9381 | 51 |
| 23.76 | 3.7418 | 71 |
| 25.00 | 3.5590 | 73 |
| 28.36 | 3.1445 | 89 |

| **Table 2B** | | |
|---|---|---|
| **° 2θ** | ***d* space (Å)** | **Iᵣₑₗ (%)** |
| 7.88 | 11.2106 | 100 |
| 8.76 | 10.0863 | 19 |
| 10.96 | 8.0661 | 21 |
| 13.12 | 6.7426 | 36 |
| 13.80 | 6.4118 | 25 |
| 14.72 | 6.0131 | 38 |
| 15.76 | 5.6186 | 17 |
| 18.60 | 4.7666 | 40 |
| 19.32 | 4.5905 | 88 |
| 20.12 | 4.4098 | 42 |
| 21.60 | 4.1109 | 37 |
| 22.00 | 4.0370 | 40 |
| 22.56 | 3.9381 | 51 |
| 23.76 | 3.7418 | 71 |
| 25.00 | 3.5590 | 73 |
| 25.84 | 3.4451 | 26 |
| 26.24 | 3.3935 | 24 |
| 28.36 | 3.1445 | 89 |
| 29.64 | 3.0115 | 27 |
| 30.56 | 2.9229 | 20 |
| 31.40 | 2.8466 | 25 |
| 31.96 | 2.7980 | 26 |
| 33.48 | 2.6744 | 15 |
| 36.80 | 2.4404 | 21 |

A third embodiment of this aspect of the invention provides for a polymorphically stable crystalline form of lesinurad (referred to in the rest of description as "form F") which is a formic acid solvate as determined by, e.g., ¹H NMR analysis or, more preferably, a formic acid hemi-solvate.

More preferably said crystalline form F shows an X-ray powder diffraction pattern that, when collected with the Kα radiation of copper (λ = 1.5408 Å), is characterized by peaks at:
v. 11.12°, 21.60°, and 26.08° ± 0.2° 2θ; or
vi. 11.12°, 21.60°, 23.40°, 26.08° and 26.64° ± 0.2° 2θ.

Even more preferably, the crystalline form F shows an X-ray powder diffraction pattern that, when collected with the Kα radiation of copper (λ = 1.5408 Å), is characterized by at least 3 peaks (± 0.2° 2θ) selected from Table 3A or 3B:

| **Table 3A** | | |
|---|---|---|
| **° 2θ** | ***d* space (Å)** | **Iᵣₑₗ (%)** |
| 7.40 | 11.9366 | 18 |
| 11.12 | 7.9504 | 100 |
| 14.52 | 6.0955 | 22 |
| 16.64 | 5.3234 | 16 |
| 17.72 | 5.0013 | 21 |
| 19.20 | 4.6190 | 18 |
| 20.40 | 4.3499 | 21 |
| 21.60 | 4.1109 | 40 |
| 22.28 | 3.9869 | 19 |
| 23.40 | 3.7986 | 39 |
| 24.32 | 3.6569 | 31 |
| 25.68 | 3.4662 | 20 |
| 26.08 | 3.4140 | 58 |
| 26.64 | 3.3435 | 34 |
| 27.76 | 3.2111 | 20 |

| **Table 3B** | | |
|---|---|---|
| **° 2θ** | ***d* space (Å)** | **Iᵣₑₗ (%)** |
| 7.40 | 11.9366 | 18 |
| 9.68 | 9.1296 | 14 |
| 11.12 | 7.9504 | 100 |
| 11.88 | 7.4434 | 15 |
| 13.32 | 6.6418 | 14 |
| 14.52 | 6.0955 | 22 |
| 15.52 | 5.7049 | 11 |
| 16.64 | 5.3234 | 16 |
| 17.72 | 5.0013 | 21 |
| 18.20 | 4.8704 | 9 |
| 19.20 | 4.6190 | 18 |
| 19.64 | 4.5165 | 13 |
| 20.40 | 4.3499 | 21 |
| 21.60 | 4.1109 | 40 |
| 22.28 | 3.9869 | 19 |
| 23.40 | 3.7986 | 39 |
| 24.32 | 3.6569 | 31 |
| 25.68 | 3.4662 | 20 |
| 26.08 | 3.4140 | 58 |
| 26.64 | 3.3435 | 34 |
| 27.76 | 3.2111 | 20 |
| 28.88 | 3.0890 | 11 |
| 29.32 | 3.0437 | 10 |
| 29.96 | 2.9801 | 14 |
| 30.48 | 2.9304 | 8 |
| 31.12 | 2.8716 | 11 |
| 32.12 | 2.7844 | 16 |
| 33.72 | 2.6559 | 10 |
| 35.12 | 2.5532 | 9 |
| 37.04 | 2.4251 | 9 |

A further embodiment of this aspect of the invention relates to a crystalline salt of lesinurad with dicyclohexylamine.

Preferably said dicyclohexylamine salt of lesinurad shows an X-ray powder diffraction pattern that, when collected with the Kα radiation of copper (λ = 1.5408Å), is characterized by peaks at:
vii. 10.36°, 11.12°, and 17.08° ± 0.2° 2θ; or
viii. 7.88°, 10.36°, 11.12°, and 17.08° ± 0.2° 2θ.

More preferably the dicyclohexylamine salt of lesinurad shows an X-ray powder diffraction pattern that, when collected with the Kα radiation of copper (λ = 1.5408Å), is characterized by at least 3 peaks (± 0.2° 2θ) selected from Table 4A or 4B:

| **Table 4A** | | |
|---|---|---|
| **°2θ** | ***d* space (Å)** | **Iᵣₑₗ (%)** |
| 7.88 | 11.2106 | 44 |
| 10.36 | 8.5319 | 49 |
| 11.12 | 7.9504 | 48 |
| 17.08 | 5.1872 | 100 |
| 19.60 | 4.5256 | 28 |
| 19.92 | 4.4536 | 26 |
| 22.00 | 4.0370 | 38 |
| 22.88 | 3.8837 | 21 |

| **Table 4B** | | |
|---|---|---|
| **° 2θ** | ***d* space (Å)** | **Iᵣₑₗ (%)** |
| 7.88 | 11.2106 | 44 |
| 10.36 | 8.5319 | 49 |
| 11.12 | 7.9504 | 48 |
| 13.08 | 6.7631 | 20 |
| 16.24 | 5.4536 | 16 |
| 17.08 | 5.1872 | 100 |
| 19.60 | 4.5256 | 28 |
| 19.92 | 4.4536 | 26 |
| 20.44 | 4.3415 | 15 |
| 22.00 | 4.0370 | 38 |
| 22.88 | 3.8837 | 21 |
| 23.64 | 3.7605 | 18 |
| 26.40 | 3.3733 | 15 |

Another embodiment of this aspect of the invention relates to a crystalline salt of lesinurad with 4-methylmorpholine.

Preferably said salt of lesinurad with 4-methylmorpholine shows an X-ray powder diffraction pattern that, when collected with the Kα radiation of copper (λ = 1.5408Å), is characterized by peaks at:
ix. 9.64°, 15.48°, 21.48°, and 22.80 ± 0.2° 2θ; or
x. 7.68°, 9.64°, 14.64°, 15.48°, 15.88°, 21.48°, and 22.80° ± 0.2° 2θ.

More preferably said 4-methylmorpholine salt of lesinurad shows an X-ray powder diffraction pattern that, when collected with the Kα radiation of copper (λ = 1.5408 Å), is characterized by at least 4 peaks (± 0.2° 2θ) selected from Table 5A or 5B:

| **Table 5A** | | |
|---|---|---|
| **° 2θ** | ***d* space (Å)** | **Iᵣₑₗ (%)** |
| 7.68 | 11.5021 | 47 |
| 8.76 | 10.0863 | 34 |
| 9.64 | 9.1674 | 63 |
| 11.28 | 7.8380 | 34 |
| 14.64 | 6.0458 | 54 |
| 15.48 | 5.7196 | 72 |
| 15.88 | 5.5764 | 47 |
| 20.72 | 4.2834 | 30 |
| 21.48 | 4.1336 | 100 |
| 22.80 | 3.8971 | 84 |
| 23.68 | 3.7543 | 38 |
| 26.92 | 3.3093 | 38 |

| **Table 5B** | | |
|---|---|---|
| **° 2θ** | ***d* space (Å)** | **Iᵣₑₗ (%)** |
| 7.68 | 11.5021 | 47 |
| 8.76 | 10.0863 | 34 |
| 9.64 | 9.1674 | 63 |
| 11.28 | 7.8380 | 34 |
| 14.64 | 6.0458 | 54 |
| 15.48 | 5.7196 | 72 |
| 15.88 | 5.5764 | 47 |
| 17.64 | 5.0238 | 22 |
| 20.72 | 4.2834 | 30 |
| 21.48 | 4.1336 | 100 |
| 22.80 | 3.8971 | 84 |
| 23.68 | 3.7543 | 38 |
| 24.12 | 3.6868 | 24 |
| 25.48 | 3.4930 | 20 |
| 26.92 | 3.3093 | 38 |
| 27.36 | 3.2571 | 23 |
| 30.32 | 2.9455 | 25 |

In a second aspect thereof, the invention refers to processes for the preparation of the crystalline forms object of the first aspect of the invention.

According to a first embodiment of this second aspect of the invention, a process for the preparation of crystalline form J is provided, said process comprising the steps of:
a) dispersing a crystalline form of lesinurad in water or in water containing an amount of acetic acid from 0.01 to 0.5% by weight;
b) maintaining the dispersion obtained in step a) under stirring at temperatures from 5 to 25 °C so as to cause precipitation of at least a portion of lesinurad in the form of crystalline form J; and
c) recovering said crystalline form J of lesinurad and, optionally, drying it;
wherein the crystalline form of lesinurad used in step a) is selected from the group comprising, or preferably consisting of, crystalline form K, crystalline form F and mixtures thereof.

Step a) entails the dispersion of a crystalline form of lesinurad (preferably selected from the group comprising, or more preferably consisting of, crystalline form F, crystalline form K and mixtures thereof) in water or in water containing an amount of acetic acid from 0.01 to 0.5% by weight, preferably from 0.05 to 0.3% by weight, even more preferably from 0.1 to 0.2% by weight. This step is preferably carried out at a temperature from 5 °C to 25 °C, more preferably from 10 °C to 23 °C, even more preferably from 15 °C to 22 °C. The amount of water optionally in mixture with acetic acid can vary in a very wide range; preferably, the overall volume of liquid may vary from 5 mL to 50 mL per gram of lesinurad; more preferably, the volume is from 10 to 40 mL, even more preferably from 20 to 30 mL per gram of lesinurad.

The following step b) includes maintaining the dispersion prepared in step a) under stirring so as to cause precipitation of at least a portion of lesinurad in the form of crystalline form J. This step is carried out by maintaining the dispersion prepared in step a) at temperatures from 5 to 25 °C (preferably from 8 °C to 23 °C, more preferably from 10 °C to 22 °C, even more preferably from 11 °C to 21 °C) so as to cause precipitation of at least a portion of lesinurad in the form of crystalline form J, preferably for at least 2 hours, more preferably for a period from 6 to 20 hours, more preferably from 10 to 15 hours.

After precipitation, the crystalline form J is recovered in step c) using known techniques such as filtration or centrifugation and optionally dried, e.g. according to the any of the procedures known in the field, preferably by treating the recovered solid at temperatures from 30 to 60 °C (more preferably from 35 to 55 °C, even more preferably from 40 to 45 °C) optionally under reduced pressure.

In a possible variant of this embodiment, an optional step a') is carried out between steps a) and b), comprising seeding the dispersion prepared in step a) with form J of lesinurad so as to promote precipitation of the desired crystalline form. Preferably, the seed is added in a weight ratio from 0.5% to 10% (preferably from 1% to 5%) with respect to the amount of lesinurad crystalline form F or K used in step a).

A second embodiment of this aspect of the invention provides for the preparation of crystalline form K of lesinurad, said process comprising the steps of:
d) dispersing lesinurad or a salt thereof in a solvent selected from the group comprising, or preferably consisting of, acetic acid, water and mixtures thereof;
e) maintaining the dispersion obtained in step d) under stirring so as to cause precipitation of at least a portion of lesinurad in the form of crystalline form K;
f) recovering said crystalline form K of lesinurad and, optionally, drying it;
wherein step e) is carried out according to step e.1) or, alternatively, the sequence of steps e.2) and e.3), reported below:
e.1) maintaining the dispersion obtained in step d) under stirring at a temperature from 10 to 30 °C for at least 30 minutes; or
e.2) heating the dispersion obtained in step d) to a temperature from 31 to 80 °C so as to obtain a mixture; and
e.3) cooling the resulting mixture to a temperature from 10 to 30 °C.

Step d) entails the dispersion of lesinurad or a salt thereof in a solvent selected from the group comprising, or preferably consisting of, acetic acid (preferably glacial acetic acid), water and mixtures thereof. Said dispersion is normally carried out at a temperature from 5 °C to 30 °C, preferably from 10 °C to 25 °C, more preferably from 15 °C to 22 °C. The amount of solvent can vary in a very wide range; preferably, the overall volume of solvent may vary from 1 mL to 30 mL per gram of lesinurad; more preferably, the volume is from 2 to 20 mL, even more preferably from 3 to 10 mL per gram of lesinurad.

The ratio between acetic acid and water in said mixtures can vary in a very wide range; preferably from 0.5:1 to 20:1 (V/V), more preferably from 1:1 to 10:1 (V/V), even more preferably from 2:1 to 4:1 (V/V).

The following step e) includes maintaining the dispersion prepared in step d) under stirring so as to cause precipitation of at least a portion of lesinurad in the form of crystalline form K. This step can be carried out according to either step e.1) or the sequence of steps e.2) and e.3).

Step e.1) comprises maintaining the dispersion obtained in step d) under stirring at temperatures from 10 to 30 °C (preferably from 15 °C to 25 °C, more preferably from 20 °C to 23 °C) for at least 30 minutes (preferably for a period from 60 minutes to 48 hours, more preferably from 5 to 24 hours).

The alternative synthetic path includes heating the dispersion obtained in step d) to temperatures from 31 to 80 °C (preferably from 40 °C to 75 °C, more preferably from 50 to 60 °C) so as to provide a mixture (according to step e.2)), preferably a solution, which is subsequently cooled to a temperature from 10 to 30 °C, preferably from 15 °C to 25 °C, more preferably from 20 °C to 23 °C (according to step e.3)).

After precipitation, the crystalline form K is recovered in step f) using known techniques such as filtration or centrifugation and optionally dried, e.g. according to the any of the procedures known in the field, preferably by treating the recovered solid at temperatures from 30 to 45 °C (more preferably from 35 to 40 °C) optionally under reduced pressure.

In a possible variant of this embodiment, a further and optional step e') is carried out between steps e.1) and f) or e.2) and e.3), comprising adding water to the masses resulting from steps e.1) or e.2) in order to increase recovery yields.

In a further variant of this embodiment of the invention, an additional and optional step e") is carried out between steps d) and e.1) or e.2) and e.3), comprising seeding the mass resulting from steps d) or e.1) with crystalline form K of lesinurad so as to promote precipitation of the desired crystalline form. Preferably, the seed is added in a weight ratio from 0.5% to 1.0% with respect to the amount of lesinurad or salt thereof used in step d) and, at a temperature from 10 to 20 °C lower than the temperature used in step e.2).

Steps e') and e") can be carried out independently of each other and in any order.

A third embodiment of this aspect of the invention provides for the preparation of crystalline form F, said process comprising the steps of:
g) dispersing lesinurad or a salt thereof in a solvent mixture comprising formic acid and water;
h) maintaining the dispersion obtained in step g) under stirring so as to cause precipitation of at least a portion of lesinurad in the form of crystalline form F; and
i) recovering said crystalline form of lesinurad and, optionally, drying it.

Step g) entails the dispersion of lesinurad or a salt thereof in a solvent mixture comprising formic acid and water, at temperatures normally from 10 °C to 35 °C, preferably from 15 °C to 30 °C, even more preferably from 20 °C to 25 °C. The amount of the mixture comprising formic acid and water can vary in a very wide range; preferably, the overall volume of solvent may vary from 1 mL to 30 mL per gram of lesinurad; more preferably, the volume is from 2 to 20 mL, even more preferably from 3 to 10 mL per gram of lesinurad.

The ratio between formic acid and water in said solvent mixtures can vary in a very wide range; preferably from 0.5:1 to 20:1 (V/V), more preferably from 1:1 to 10:1 (V/V), even more preferably from 2:1 to 4:1 (V/V).

The following step h) includes maintaining the dispersion prepared in step g) under stirring so as to cause precipitation of at least a portion of lesinurad in the form of solvate form F. This step is normally carried out at a temperature from 10 to 45 °C (preferably from 15 °C to 40 °C, more preferably from 20 to 35 °C) for at least 2 hours, or preferably for a period from 3 to 48 hours, more preferably from 10 to 24 hours.

After precipitation, the crystalline form F is recovered using known techniques such as filtration or centrifugation and optionally dried, e.g. according to the any of the procedures known in the field, preferably by treating the recovered solid at temperatures from 30 to 45 °C (preferably from 35 to 40 °C) optionally under reduced pressure.

In a possible variant of this embodiment, a further and optional step g') is carried out between steps g) and h), comprising adding water to the dispersion resulting from step g) in order to increase the recovery yields.

Forms of lesinurad suitable to be used in steps d) and g) are for example selected from the group comprising, or preferably consisting of, lesinurad forms 1 or 2 (as disclosed in WO 2012/092395 A2), forms III, IV, V, and VI (as disclosed in WO 2015/075561 A2), its amorphous form (as disclosed in WO 2012/092395 A2) and mixtures thereof. Salts of lesinurad suitable for the aim include, e.g., its sodium and potassium salt (as disclosed in WO 2009/070740 A2), its calcium, hydrochloride, esylate, mesylate, 1,2-ethanedisulfonate, 1,2-ethanedisulfonate, isethionate, and arginine salt (as disclosed in WO 2015/095703 A2) or preferably its dicyclohexylamine and 4-methylmorpholine salts.

A further embodiment of this aspect of the invention provides for the preparation of a dicyclohexylamine or 4-methylmorpholine salt of lesinurad, said process comprising the steps of:
j) dispersing lesinurad in at least one solvent;
k) mixing the dispersion obtained in step j) with dicyclohexylamine or 4-methylmorpholine so as to obtain a mixture;
I) maintaining the resulting mixture under stirring so as to cause precipitation of the dicyclohexylamine or of the 4-methylmorpholine salt of lesinurad; and
m) recovering said dicyclohexylamine or 4-methylmorpholine salt of lesinurad and, optionally, drying it.

Forms of lesinurad suitable to be used in step j) are for example selected from the group comprising, or preferably consisting of, lesinurad forms 1 or 2 (as disclosed in WO 2012/092395 A2), forms III, IV, V, and VI (as disclosed in WO 2015/075561 A2), its amorphous form (as disclosed in WO 2012/092395 A2) and mixtures thereof.

Step j) entails the dispersion of lesinurad in at least one solvent (preferably dichloromethane) at a temperature normally from 10 °C to 35 °C, preferably from 15 °C to 30 °C, even more preferably from 20 °C to 25 °C. The amount of solvent can vary in a very wide range; preferably, the overall volume of solvent may vary from 1 mL to 30 mL per gram of lesinurad; more preferably, the volume is from 2 to 20 mL, even more preferably from 3 to 10 mL per gram of lesinurad.

According to the following step k) the dispersion obtained in step j) is mixed with dicyclohexylamine or 4-methylmorpholine. This step is preferably carried out by adding the dicyclohexylamine or 4-methylmorpholine (optionally solubilized in a suitable solvent) to the dispersion of lesinurad obtained in step j). Said addition can be carried out in a single step (i.e. a single addition of the entire amount to be added) or, in multiple additions (i.e. by dosing the quantity to be added).

Step I) includes maintaining the mixture prepared in step k) under stirring so as to cause precipitation of dicyclohexylamine or of the 4-methylmorpholine salt of lesinurad. This step is normally carried out at a temperature from 10 to 45 °C (preferably from 15 °C to 40 °C, more preferably from 20 to 35 °C) for at least 10 minutes, or preferably for a period from 20 minutes to 5 hours, more preferably from 30 to 1 hour.

After precipitation, the dicyclohexylamine or the 4-methylmorpholine salt of lesinurad are recovered using known techniques such as filtration or centrifugation and optionally dried, e.g. according to the any of the procedures known in the field, preferably by treating the recovered solids at temperatures from 30 to 55 °C (preferably from 35 to 45 °C) optionally under reduced pressure.

The novel polymorphic forms described in the present application are stable towards stressed conditions. Moreover, they can be prepared with processes cheap and effective, particularly suitable for large scale production. These processes can be used to obtain lesinurad with a chemical purity determined by HPLC greater than 99.5%, particularly suitable for the preparation of pharmaceutical dosage forms.

The crystalline forms of the present invention (preferably the crystalline forms F, J and K of lesinurad) can be used, in admixture with one or more pharmaceutically acceptable excipients, for the preparation of pharmaceutical formulations such as tablets, capsules, pills, powders, granules, pastilles, lozenges, elixirs, syrups, solutions, suspensions, emulsions, drops, lotions, sprays, tinctures, creams, pomades, gels, ointments, suppositories and transdermal devices for oral, enteral, parenteral or topical administration.

In a further aspect thereof, the present invention relates to crystalline forms F, J and K of lesinurad, to the pharmaceutical compositions comprising them (preferably to the pharmaceutical compositions comprising crystalline form J of lesinurad) for use in therapy (preferably for the treatment of gout).

### Examples

**XRPD:** Analyses were performed on an EasyX600 TNX bench-top diffractometer at 25 °C, using a Cu Kα tube (30 kV, 20 mA, λ = 1.5408 Å) as the X-ray source, equipped with a scintillation detector. Data collection was made in coupled mode and in theta-theta configuration, with 2θ range increment of 0.04°. Samples were accurately grinded and placed in the hollow of a spinning aluminum sampler. The instrument was previously calibrated by means of zinc oxide, then allowing collection and elaboration of data by means of DFP acquisition software. RH (relative humidity) in the cabin: 28-30%.

**NMR:** ¹H NMR solution spectra were performed on a Jeol Eclipse 300 at 298 K, using DMSO-d₆ as solvent. Chemical shifts are measured in δ ppm relative to tetramethylsilane. Accurately weighted amounts of sample were dissolved in a suitable test NMR tube and analysed with 10 seconds delay.

**Karl Fischer titration:** The water content was determined by means of a Mettler-Toledo V20 volumetric K.F. titrator, using K.F. reagent one component as titrating solution (1 ml correspond to 2 mg of water) and dry methanol as titration medium.

### Example 1. Preparation of crystalline form K of lesinurad.

8.8 grams of lesinurad Form IV (prepared according to WO 2015/075561 A2) were suspended under stirring in 26.4 mL of acetic acid at 20-25 °C in a glass flask equipped with thermometer, magnetic stirrer and condenser. The resulting suspension was heated to 70 °C, in order to obtain a clear solution, and 6.6 mL of water were added thereto. The mixture was then cooled to 25 °C thus causing lesinurad to precipitate. The obtained solid was filtered and dried at 40 °C under reduced pressure, thus affording 6.6 grams of crystalline form K of lesinurad (final water content, as per Karl Fisher titration, 0.2% w/w).

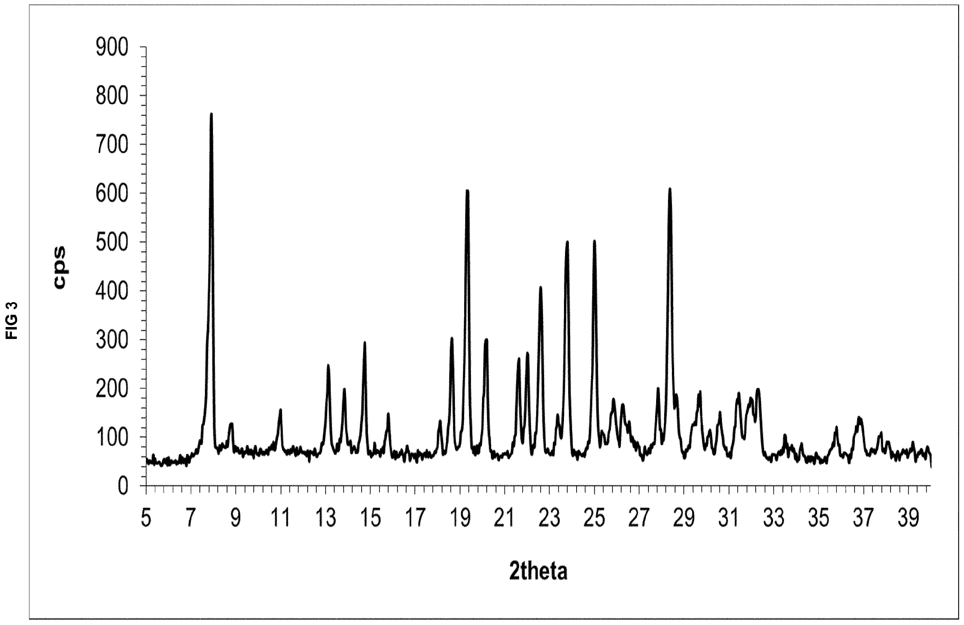

The obtained product was analysed by XRPD, obtaining the diffractogram shown in Figure 3.

A ¹H NMR analysis of a portion of the product was carried out for estimating the amount of residual acetic acid in the sample, which was found to be of about 13% by weight (corresponding to a mono-solvate).

### Example 2. Preparation of crystalline form K of lesinurad.

8.8 grams of lesinurad Form IV (prepared according to WO 2015/075561 A2) were suspended under stirring in 26.4 mL of acetic acid at 20-25 °C in a glass flask equipped with thermometer, and magnetic stirrer. The resulting suspension was maintained under stirring at the same temperature for 12 hours. The obtained solid was filtered and dried at 40 °C under reduced pressure, thus affording 6.8 grams of crystalline form K of lesinurad (final water content, as per Karl Fisher titration, 0.2% w/w).

The obtained product was analysed by XRPD and ¹H NMR, obtaining a diffractogram and a spectrum corresponding to those obtained in example 1.

### Example 3. Preparation of crystalline form F of lesinurad.

6.2 grams of lesinurad Form IV (prepared according to WO 2015/075561 A2) were suspended under stirring and at 20-25 °C in 18.3 mL of formic acid and 5.0 mL of water. The resulting suspension was maintained under stirring at 30-35 °C for 12 hours. The obtained solid was filtered and dried at 40 °C under reduced pressure, thus affording 3.2 grams of crystalline form F of lesinurad (final water content, as per Karl Fisher titration, 0.8% w/w).

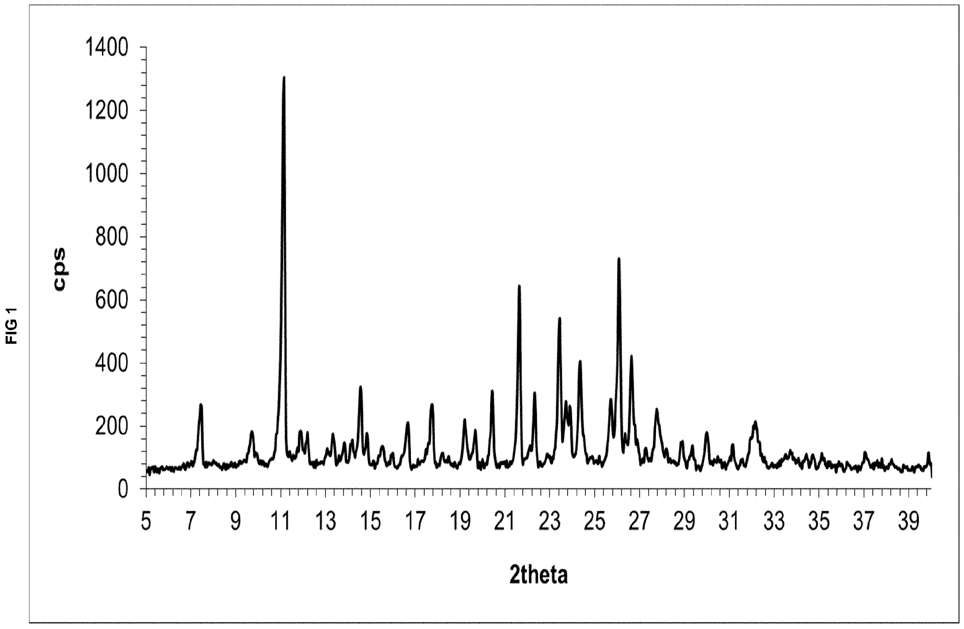

The obtained product was analysed by XRPD, obtaining the diffractogram shown in Figure 1.

A ¹H NMR analysis of a portion of the product was carried out for estimating the amount of residual formic acid in the sample, which was found to be of about 5.1% by weight (corresponding to a hemi-solvate).

### Example 4. Preparation of crystalline form J of lesinurad.

2.7 grams of lesinurad Form K (prepared in according to example 1) were suspended at 20°C and under stirring in 30.0 mL of water containing 0.3 g of acetic acid. The resulting suspension was maintained under stirring at the same temperature for 16 hours. The obtained solid was filtered and dried at 40 °C under reduced pressure, thus affording 2.2 grams of crystalline form J of lesinurad (final water content, as per Karl Fisher titration, <0.1% w/w).

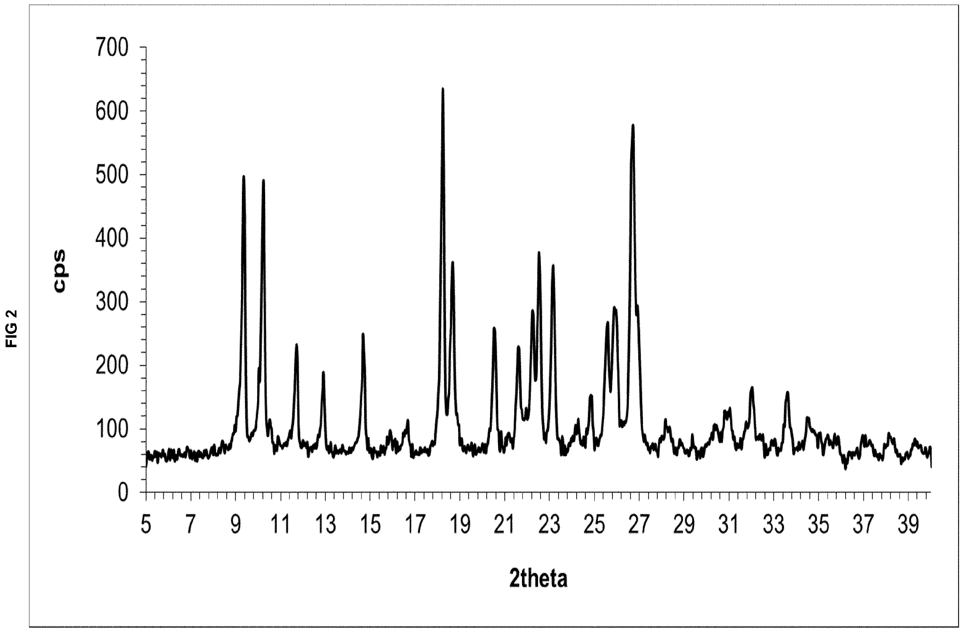

The obtained product was analysed by XRPD, obtaining the diffractogram shown in Figure 2.

### Example 5. Thermal stability of the crystalline form K of lesinurad.

Crystalline form K (2 g), prepared as described in Example 1, was maintained at 50 °C under vacuum for 24 hours. Then it was cooled to room temperature and subjected to XRPD analysis, giving rise to a XRPD spectrum corresponding to the one obtained in example 1.

### Example 6. Thermal stability of the crystalline form F of lesinurad

Crystalline form F (2 g), prepared as described in Example 3, was maintained at 35 °C under vacuum for 24 hours. Then it was cooled to room temperature and subjected to XRPD analysis, giving rise to a XRPD spectrum corresponding to the one obtained in example 3.

### Example 7. Thermal stability of the crystalline form J of lesinurad.

Crystalline form J (2 g), prepared as described in Example 4, was maintained at 80 °C under atmospheric pressure for 24 hours. Then it was cooled to room temperature and subjected to XRPD analysis, giving rise to a XRPD spectrum corresponding to the one obtained in example 4.

### Example 8. Preparation of lesinurad dicyclohexylamine salt.

Water (7.8 mL) and a 32% (w/w) aqueous solution of sodium hydroxide (3.94 g, 31.54 mmol) were subsequently added to a solution of methyl 2-((5-bromo-4-(4-cyclopropylnaphthalen-1-yl)-4*H*-1,2,4-triazol-3-yl)thio)acetate (7.78 g, 18.60 mmol) in dichloromethane (70.0 mL) so as to maintain an internal temperature lower than 30 °C. The mixture was then stirred at 25-30 °C (maintaining a pH ≥ 10) while monitoring by HPLC and considered complete when the content of the starting material was lower than 0.1%. Water (23.0 mL) and hydrochloric acid 36% (1.12 g, 30.69 mmol) were added thereto so as to obtain a pH of the aqueous layer between 5 and 7. After having separated the layers, dicyclohexylamine (3.57 g, 19.72 mmol) was added to the organic phase and the resulting mixture maintained under stirring at 25-30 °C for 10 minutes. The solution was filtered on a celite/charcoal pad and concentrated under reduced pressure up to obtain a semisolid residue to which isopropanol (23.5 mL) was added. The mixture was heated to reflux up to obtain an internal temperature of between 75 and 80 °C, maintained under stirring at the same temperature for 3 hours then cooled to 0 °C. The resulting solid was filtered, washed with isopropanol and dried at 35 °C under reduced pressure, thus affording 7.62 g of the dicyclohexylamine salt of lesinurad.

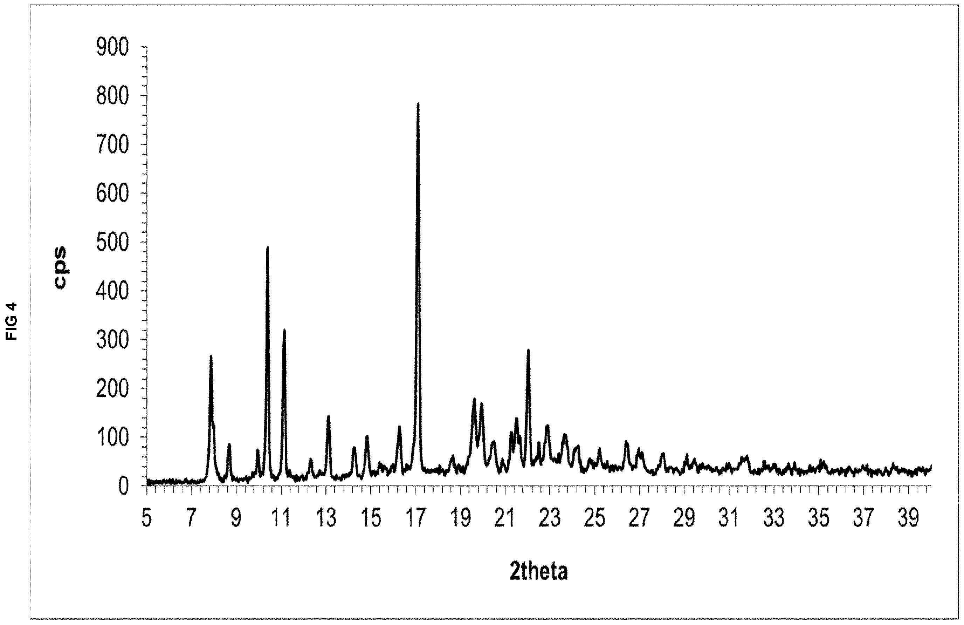

The obtained product was analysed by XRPD, obtaining the diffractogram shown in Figure 4.

### Example 9. Preparation of lesinurad 4-methylmorpholine salt.

Water (7.8 mL) and a 32% (w/w) aqueous solution of sodium hydroxide (3.94 g, 31.54 mmol) were subsequently added to a solution of methyl 2-((5-bromo-4-(4-cyclopropylnaphthalen-1-yl)-4*H*-1,2,4-triazol-3-yl)thio)acetate (7.78 g, 18.60 mmol) in dichloromethane (70.0 mL) so as to maintain an internal temperature lower than 30 °C. The mixture was then stirred at 25-30 °C (maintaining a pH ≥ 10) while monitoring by HPLC and considered complete when the content of the starting material was lower than 0.1%. Water (23.0 mL) and hydrochloric acid 36% (1.12 g, 30.69 mmol) were added thereto so as to obtain a pH of the aqueous layer between 5 and 7. After having separated the layers, 4-methylmorpholine (1.99 g, 19.72 mmol) was added to the organic phase and the resulting mixture maintained under stirring at 25-30 °C for 10 minutes. The solution was filtered on a celite/charcoal pad and concentrated under reduced pressure up to obtain a semisolid residue to which isopropanol (23.5 mL) was added. The mixture was heated to reflux up to obtain an internal temperature of between 75 and 80 °C, maintained under stirring at the same temperature for 3 hours then cooled to 0 °C. The resulting solid was filtered, washed with isopropanol and dried at 35 °C under reduced pressure, thus affording 6.11 g of the 4-methylmorpholine salt of lesinurad.

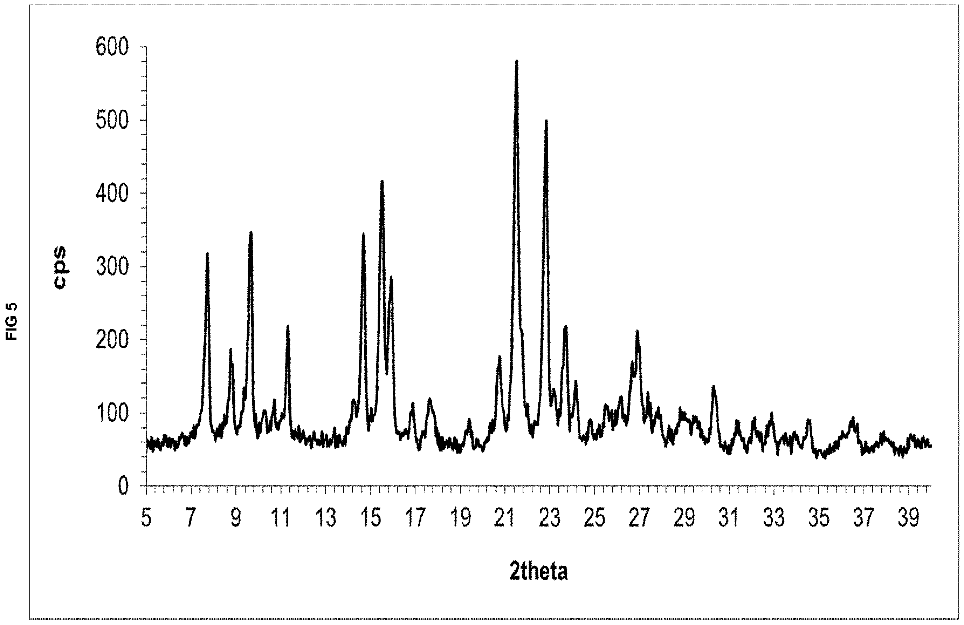

The obtained product was analysed by XRPD, obtaining the diffractogram shown in Figure 5.

### Example 10. Preparation of crystalline form K of lesinurad.

Lesinurad dicyclohexylamine salt (6.11 g), prepared as described in Example 8, was suspended at 20-25 °C and under stirring in a 2:1 (V/V) mixture of acetic acid and water (18.3 mL). The resulting suspension was seeded with crystalline form K, maintained under stirring at the same temperature for 24 hours and finally cooled to 0 °C. The obtained solid was filtered, washed with a 2:1 (V/V) mixture of acetic acid and water and dried at 40 °C under reduced pressure, thus affording 4.6 grams of crystalline form K of lesinurad.

The obtained product was analysed by XRPD and ¹H NMR, obtaining a diffractogram and a spectrum corresponding to those obtained in example 1.

### Example 11. Preparation of crystalline form K of lesinurad.

8.5 grams of lesinurad amorphous form (prepared according to WO 2016/203436 A1) were suspended under stirring in 17.6 mL of acetic acid at 20-25 °C in a glass flask equipped with thermometer, magnetic stirrer and condenser. The resulting suspension was maintained under stirring at the same temperature for 12 hours. The obtained solid was filtered and dried at 40 °C under reduced pressure, thus affording 6.5 grams of crystalline form K of lesinurad.

The obtained product was analysed by XRPD and ¹H NMR, obtaining a diffractogram and a spectrum corresponding to those obtained in example 1.

### Example 12. Preparation of crystalline form J of lesinurad.

2.7 grams of lesinurad Form F (prepared in according to example 3) were suspended at 16 °C and under stirring in 30.0 mL of water containing 0.3 g of acetic acid. The resulting suspension was maintained under stirring at the same temperature for 16 hours. The obtained solid was filtered and dried at 40 °C under reduced pressure, thus affording 2.2 grams of crystalline form J of lesinurad (final water content, as per Karl Fisher titration, <0.1% w/w).

The obtained product was analysed by XRPD, obtaining a diffractogram corresponding to the one obtained in example 4.

### Example 13. Preparation of crystalline form F of lesinurad.

Lesinurad dicyclohexylamine salt (6.11 g), prepared as described in Example 8, was dispersed at 20-25 °C and under stirring in 18.3 mL of formic acid and 10.0 mL of water. The resulting dispersion maintained under stirring at the same temperature for 3 hours and finally cooled to 0 °C. The obtained solid was filtered, washed with a 2:1 (V/V) mixture of formic acid and water and dried at 35 °C under reduced pressure, thus affording 4.6 grams of crystalline form F of lesinurad.

The obtained product was analysed by XRPD and ¹H NMR, obtaining a diffractogram and a spectrum corresponding to those obtained in example 3.

## Claims

1. Crystalline form J of lesinurad having an X-ray powder diffraction pattern that, when collected with the Kα radiation of copper (λ = 1.5408 Å), is **characterized by** at least the following peaks at 9.32°, 15.88°, 18.20° and 26.68° ± 0.2° 2θ.

2. The crystalline form according to claim 1 wherein the X-ray powder diffraction pattern further comprises peaks at 10.20°, 18.64°, 22.24°, 22.52°, 23.12° and 25.92° ± 0.2° 2θ.

3. Crystalline form K of lesinurad having an X-ray powder diffraction pattern that, when collected with the Kα radiation of copper (λ = 1.5408 Å), is **characterized by** at least the following peaks at 7.88°, 10.96°, 19.32° and 28.36° ± 0.2° 2θ.

4. The crystalline form according to claim 3 which is an acetic acid solvate.

5. Crystalline form F of lesinurad having an X-ray powder diffraction pattern that, when collected with the Kα radiation of copper (λ = 1.5408 Å), is **characterized by** at least the following peaks at 11.12°, 21.60°, and 26.08° ± 0.2° 2θ.

6. The crystalline form according to claim 5 which is a formic acid solvate.

7. Crystalline dicyclohexylamine salt of lesinurad having an X-ray powder diffraction pattern that, when collected with the Kα radiation of copper (λ = 1.5408 Å), is **characterized by** at least the following peaks at 10.36°, 11.12°, and 17.08° ± 0.2° 2θ.

8. Crystalline 4-methylmorpholine salt of lesinurad having an X-ray powder diffraction pattern that, when collected with the Kα radiation of copper (λ = 1.5408 Å), is **characterized by** at least the following peaks at 9.64°, 15.48°, 21.48°, and 22.80 ± 0.2° 2θ.

9. Process for the preparation of a crystalline form J of lesinurad as defined in any one of claims 1 and 2, said process comprising the steps of:
a) dispersing a crystalline form of lesinurad in water or in water containing an amount of acetic acid from 0.01 to 0.5% by weight;
b) maintaining the dispersion obtained in step a) under stirring at temperatures from 5 to 25 °C so as to cause precipitation of at least a portion of lesinurad in the form of crystalline form J;
c) recovering said crystalline form J of lesinurad and, optionally, drying it;
wherein the crystalline form of lesinurad used in step a) is selected from the group consisting of crystalline form F, as defined in any one of claims 5 and 6, crystalline form K, as defined in any one of claims 3 and 4, and mixtures thereof.

10. Process for the preparation of a crystalline form K of lesinurad as defined in any one of claims 3 and 4, said process comprising the steps of:
d) dispersing lesinurad or a salt thereof in a solvent selected from the group comprising acetic acid, water and mixtures thereof;
e) maintaining the dispersion obtained in step d) under stirring so as to cause precipitation of at least a portion of lesinurad in the form of crystalline form K;
f) recovering said crystalline form K of lesinurad and, optionally, drying it; wherein step e) is carried out according to step e.1) or, alternatively, the sequence of steps e.2) and e.3) reported below:
e.1) maintaining the dispersion obtained in step d) under stirring at a temperature from 10 to 30 °C for at least 30 minutes; or
e.2) heating the dispersion obtained in step d) to a temperature from 31 to 80 °C so as to obtain a mixture; and
e.3) cooling the resulting mixture to a temperature from 10 to 30 °C.

11. Process for the preparation of a crystalline form F of lesinurad as defined in any one of claims 5 and 6, said process comprising the steps of:
g) dispersing lesinurad or a salt thereof in a solvent mixture comprising formic acid and water;
h) maintaining the dispersion obtained in step g) under stirring so as to cause precipitation of at least a portion of lesinurad in the form of crystalline form F; and
i) recovering said crystalline form of lesinurad and, optionally, drying it.

12. The process according to any one of claims 10 and 11 wherein the salt of lesinurad used in step d) or g) is selected from the group consisting of the dicyclohexylamine salt of lesinurad defined in claim 7, the 4-methylmorpholine salt defined in claim 8, and mixtures thereof.

13. Process for the preparation of a salt of lesinurad as defined in any one of claims 7 and 8, said process comprising the steps of:
j) dispersing lesinurad in at least one solvent;
k) mixing the dispersion obtained in step j) with dicyclohexylamine or 4-methylmorpholine so as to obtain a mixture;
I) maintaining the resulting mixture under stirring so as to cause precipitation of the dicyclohexylamine or of the 4-methylmorpholine salt of lesinurad;
m) recovering said dicyclohexylamine or 4-methylmorpholine salt of lesinurad and, optionally, drying it.

14. Pharmaceutical compositions comprising, as active ingredient, an effective amount of the crystalline form as defined in any one of claims 1 to 8.

15. Crystalline forms as defined in any one of claims 1 to 8 or pharmaceutical compositions as defined in claim 14 for use in therapy.
